# EUROPÄISCHE PATENTSCHRIFT

(11) **EP 0 519 943 B1**
(45) Veröffentlichungstag und Bekanntmachung des Hinweises auf die Patenterteilung: **11.01.1995**
(21) Anmeldenummer: 91905079.9
(22) Anmeldetag: 04.03.1991
(51) Int. Cl.: C11D 3/48

(54) **REINIGUNGS- UND DESINFEKTIONSMITTEL**
CLEANING AND DISINFECTING AGENT
AGENT DE NETTOYAGE ET DE DESINFECTION

(30) Priorität: 12.03.1990 DE 4007758
(43) Veröffentlichungstag der Anmeldung: 30.12.1992
(73) Patentinhaber: Henkel Kommanditgesellschaft auf Aktien, 40191 Düsseldorf (DE)
(72) Erfinder: HACHMANN, Klaus, Dr., D-4010 Hilden (DE); DISCH, Karlheinz, Dr., D-5657 Haan (DE); BANSEMIR, Klaus-Peter, Dr., D-4018 Langenfeld (DE); SCHWIDDEN, Hubert, D-4000 Düsseldorf 13 (DE); KAUPP, Stephanie, D-4000 Düsseldorf 13 (DE); FRIESE, Carsten, Dr., D-4010 Hilden (DE); LEHMANN, Rudolf, Dr., D-5653 Leichlingen 1 (DE); LEINEN, Hans-Theo, Dr., D-4000 Düsseldorf 1 (DE)
(86) Internationale Anmeldenummer: EP9100400
(87) Internationale Veröffentlichungsnummer: WO9113965

(56) Entgegenhaltungen:
- EP-A- 0 156 275

## Beschreibung

Die vorliegende Erfindung betrifft ein Mittel zur Reinigung und Desinfektion von Gegenständen aus medizinischen Einrichtungen und ein Verfahren unter Verwendung eines derartigen Mittels in einer automatisch betriebenen Anlage zur Sprühreinigung und Sprühdesinfektion medizinischer Einrichtungsgegenstände.

Zur Reinigung und Desinfektion von Einrichtungsgegenständen zum Beispiel von Bettgestellen, werden in Krankenhäusern üblicherweise automatisch arbeitende, programmgesteuerte Waschanlagen verwendet. Diese bestehen in ihrem wesentlichen Teil aus einer geschlossenen Kammer, in der die zu reinigenden Gegenstände mit einer oder mehreren Reinigungs- und/oder Desinfektionslösungen, die in der Regel erhöhte Temperaturen von normalerweise über 50 °C aufweisen, besprüht werden. Die Reinigungs- und Desinfektionsmittellösungen, die üblicherweise durch Auflösen von festen oder flüssigen Konzentraten in Wasser und Verdünnen auf die gewünschte Anwendungskonzentration hergestellt werden, befinden sich bei derartigen Anlagen in normalerweise beheizten Vorratsbehältern und werden automatisch durch Düsen in die Reinigungskammer gepumpt. Die Einwirkungszeit der einzelnen Lösungen auf die zu reinigenden und desinfizierenden Gegenstände ist relativ kurz und liegt in der Regel bei jeweils 30 Sekunden bis 3 Minuten. Man unterscheidet Waschanlagen, die nach dem Umwälzverfahren die Waschlaugen mehrfach benutzen, von den sogenannten Frischwasseranlagen, die für jeden zu reinigenden Gegenstand frisch angesetzte Waschlaugen verwenden, die nach einmaligem Gebrauch verworfen werden und entsorgt werden müssen.

In Frischwasseranlagen werden die zu desinfizierenden Gegenstände durch Aufsprühen einer üblicherweise erwärmten Reinigungslösung gereinigt, danach gegebenenfalls durch Aufsprühen von Wasser, dem ein Klarspülmittel zugesetzt worden sein kann, abgespült, anschließend durch Aufsprühen eines Desinfektionsmittels desinfiziert und in einem abschließenden Schritt getrocknet. Reinigungs- und Desinfektionsschritt sind bei derartigen Anlagen zeitlich getrennt und folgen durch Verwendung unterschiedliche Mittel enthaltender Sprühlösungen direkt aufeinander oder sind durch einen zwischengeschalteten Klarspülschritt voneinander getrennt.

Umwälzanlagen dagegen verwenden die Waschflotte über längere Zeit, in der Regel einen Tag, immer wieder, so daß mit der gleichen Lösung mehrere, normalerweise über 100 zu reinigende Einheiten gereinigt werden können. Da in derartigen Umwälzanlagen meist nur eine Flotte verwendet wird, muß diese sowohl reinigende als auch desinfizierende Wirkung haben. Falls die nach dem Umwälzverfahren arbeitenden Anlagen einen Klarspülschritt aufweisen, wird die in diesem Schritt verwendete Klarspüllösung nach Gebrauch entweder verworfen oder gelangt in die umlaufende Reinigungsflotte, die dadurch zunehmend verdünnt wird, was besonders hohe Anforderungen an die Reinigungs- und Desinfektionsleistung des in der Waschlauge verwendeten Mittels stellt. In dem Fall der Vereinigung von gebrauchter Reinigungs- und Klarspüllösung erfolgt normalerweise ein automatischer, vom Füllstand des Vorratsbehälters abhängiger Abfluß eines Teils der verdünnten Reinigungsflotte, die dann durch gegebenenfalls automatische Zudosierung von Frischwasser und/oder Reinigungsmittelkonzentrat ergänzt werden kann.

Ein Reinigungs- und Desinfektionsmittel muß, um in den vorstehend beschriebenen automatischen Anlagen, insbesondere in den nach dem Umwälzverfahren arbeitenden, verwendet werden zu können, eine Reihe von Bedingungen erfüllen. So muß es eine gute Reinigungsleistung bei gleichzeitiger guter Desinfektionsleistung aufweisen, die darüberhinaus beide innerhalb kurzer Zeit zur Wirkung kommen müssen. Da die Flotte in den automatischen Anlagen bei erhöhter Temperatur mit hohem Druck versprüht wird, muß es möglichst schaumfrei sein und darf gegenüber allen in den zu reinigenden Gegenständen verarbeiteten Materialien nicht korrosiv sein. Außerdem muß es verträglich mit den Bestandteilen eines eventuell ebenfalls verwendeten Klarspülmittels sein, das insbesondere bei Umwälzanlagen laufend in die Reinigungsflotte gelangen kann. Die Anwendungslösung des Mittels muß über längere Zeit, mindestens einen Tag, bei höheren Temperaturen, mindestens 50 °C, stabil sein und das Mittel sollte in der Anwendung wie auch der nachfolgenden Entsorgung eine möglichst hohe Umweltverträglichkeit aufweisen.

Bisher übliche Reinigungs- und Desinfektionsmittel, besonders solche für nach dem Umwälzverfahren arbeitende Anlagen, weisen normalerweise einen sauren pH-Wert auf, enthalten in der Regel Desinfektionsmittel mit flüchtigen Bestandteilen, zum Beispiel aus der Substanzklasse der aliphatischen Aldehyde, und erfüllen die an Reinigungs- und Desinfektionsmittel für automatische Anlagen zu stellenden Bedingungen nur unvollkommen, da insbesondere bei erhöhten Temperaturen ihre Verwendung zu Geruchsbelästigungen durch die Abluft der Reinigungsanlage führen kann.

Es bestand daher die Aufgabe, über einen längeren Zeitraum bei erhöhter Temperatur stabile, mikrobiologisch wirksame Sprühreinigungs- und -desinfektionsmittel zu entwickeln, die weitgehend pH-neutral, möglichst wenig korrosiv, umweltverträglich und unter den beschriebenen Anwendungsbedingungen möglichst schaumfrei sind.

Die erfindungsgemäßen Reinigungsmittel, die eine mindestens binäre Desinfektionsmittelkombination vorzugsweise aus bestimmten Glutaminsäurederivaten und quartären Ammoniumverbindungen, ein oder mehrere schaumarme Tenside, vorzugsweise aus den Gruppen der nichtionischen Tenside und der Amphotenside, und gegebenenfalls einen oder mehrere Komplexbildner, insbesondere aus der Gruppe der Phosphono- und Aminocarbonsäuren, und/oder weitere Desinfektionsmittel, vorzugsweise aus der Klasse der quartären Phosphoniumverbindungen, sowie ein oder mehrere organische Lösungsmittel, insbesondere aus den Gruppen der niederen Alkohole, Glykole und Glykolether, enthalten, sind unter den Anwendungsbedingungen schaumfrei, über einen längeren Zeitraum stabil, mikrobiologisch wirksam, pH-neutral und nicht korrosiv und erfüllen die an Reinigungs- und Desinfektionsmittel für die automatische Sprühreinigung und Sprühdesinfektion, insbesondere unter Einsatz von Umwälzanlagen, zu stellenden Anforderungen in überzeugender Weise.

Die erfindungsgemäßen Reinigungs- und Desinfektionsmittel sind Konzentrate nach dem Anspruch 1, die durch Auflösen in Wasser und Verdünnen auf die gewünschte Anwendungskonzentration, die vorzugsweise bei 0,5 bis 2 Gew.-% Reinigungsmittel liegt, zur Verwendung in automatischen Sprühreinigungsanlagen geeignete Lösungen ergeben.

Die nachfolgend angegebenen Mengenanteile und -bereiche in Gew.-% beziehen sich, wenn nicht anders angegeben, auf das gesamte Reinigungs- und Des infektionsmittel.

Die erfindungsgemäßen Mittel enthalten vorzugsweise eine binäre Kombination von antimikrobiellen Wirkstoffen A und B. Wirkstoff A wird ausgewählt aus den Produkten, die nach einem Verfahren, wie es in der europäischen Patentschrift EP 156 275 beschrieben ist, aus der Reaktion eines N-substiuierten Propylendiamins nach Formel (I),

R¹-NH-(CH₂)₃-NH₂ (I),

in der R¹ für einen Alkylrest mit 8 bis 18 C-Atomen, vorzugsweise 12 bis 14 C-Atomen, steht, mit Glutaminsäure oder Glutaminsäurederivaten gemäß Formel (II),

R²-O-CO-(CH₂)₂-CH(NH₂)-COOH (II),

in der R² Wasserstoff oder einen Alkylrest mit 1 bis 4 Kohlenstoffatomen bedeutet, erhältlich sind, wobei das molare Verhältnis der Reaktionspartner (I) und (II) 1:1 bis 1:2 beträgt und die Reaktion unter Austritt von Wasser und/oder Alkohol bei Temperaturen von 60 bis 175 °C über einen Zeitraum von 30 Minuten bis 10 Stunden durchgeführt wird. Diese Produkte können gegebenenfalls ethoxyliert und/oder propoxyliert sowie unter Salzbildung mit organischen oder anorganischen Säuren umgesetzt worden sein. Sie werden als alleinige antimikrobielle Wirkstoffe tensidhaltiger Reinigungsmittelkonzentrate beschrieben.

Die Herstellung N-substituierter Propylendiamine nach Formel (I) kann nach dem in der deutschen Auslegeschrift 12 72 927 beschriebenen Verfahren erfolgen. Die Herstellung von Glutaminsäure-5-estern nach Formel (II) ist zum Beispiel in den deutschen Auslegeschriften 21 58 562 oder 12 54 635 oder der deutschen Offenlegungsschrift 14 93 991 beschrieben.

Bei dem antimikrobiellen Wirkstoff B handelt es sich um eine quartäre Ammoniumverbindung nach Formel (III), 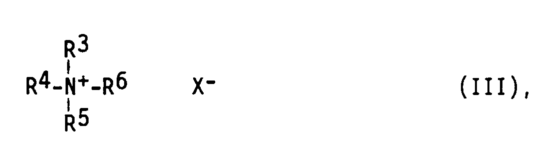
in der R³ und R⁴ unabhängig voneinander Alkylreste mit jeweils 1 bis 3 C-Atomen oder Benzyl-, halogenierte oder alkylierte Benzylreste, R⁵ und R⁶ unabhängig voneinander Alkyl- oder Benzyl-, halogenierte oder alkylierte Benzylreste mit jeweils 6 bis 22 C-Atomen und X⁻ ein Anion vorzugsweise aus den Gruppen der Halogenide oder Carboxylate bedeutet. Beispiele hierfür sind Dimethyl-dioctyl-ammoniumchlorid, Didecyl-dimethyl-ammoniumchlorid, Didodecyl-dimethyl-ammoniumchlorid, Dimethyl-ditetradecyl-ammoniumchlorid, Dihexadecyl-dimethyl-ammoniumchlorid, Dimethyl-dioctadecyl-ammoniumchlorid, Decyl-dimethyl-octyl-ammoniumchlorid, Dimethyl-dodecyl-octylammoniumchlorid, Benzyl-decyl-dimethyl-ammoniumchlorid, Benzyl-dimethyl-dodecyl-ammoniumchlorid, Benzyl-dimethyl-tetradecylammoniumchlorid, Decyl-dimethyl-(ethylbenzyl)-ammoniumchlorid, Decyl-dimethyl-(dimethyl-benzyl)-ammoniumchlorid, (Chlorbenzyl)-decyl-dimethyl-ammoniumchlorid, Decyl-(dichlorbenzyl)-dimethyl-ammoniumchlorid, Benzyl-didecyl-methyl-ammoniumchlorid, Benzyl-didodecyl-methyl-ammoniumchlorid, Benzyl-ditetradecyl-methyl-ammoniumchlorid, Benzyl-dodecyl-ethyl-methyl-ammoniumchlorid und die entsprechenden Verbindungen, die als Anionen statt Chlorid Acetat oder Propionat enthalten.

Wirkstoff A kann aber auch mit einem der antimikrobiellen Wirkstoffe C kombiniert werden oder zur zusätzlichen Verstärkung der antimikrobiellen Wirksamkeit können die bevorzugten erfindungsgemäßen Desinfektionsmittelkombinationen A und B weitere antimikrobielle Wirkstoffe C, wie quartäre Phosphoniumverbindungen, Phenole oder Biguanidverbindungen, nicht jedoch Aldehyde enthalten. Zu diesen antimikrobiellen Wirkstoffen C gehören zum Beispiel o-Phenylphenol, p-Chlor-m-kresol, 2,4-Dichlorphenol, Tetra-(hexamethylenbiguanid-hydrochlorid) und 1,1'-Hexamethylenbis-5-(4-chlorphenylbiguanid). Bevorzugte antimikrobielle Wirkstoffe sind die aus der Gruppe der quartären Phosphoniumverbindungen nach Formel (IV), 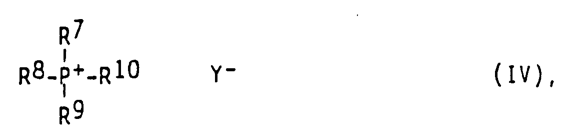
in der R⁷, R⁸, R⁹ und R¹⁰ unabhängig voneinander Alkyl-, Alkenyl-, Aryl- oder Alkylarylreste mit jeweils 1 bis 16 C-Atomen und Y⁻ ein Anion vorzugsweise aus den Gruppen der Halogenide oder Carboxylate, insbesondere Acetat oder Propionat, bedeutet, ausgewählten Verbindungen. In Frage kommen insbesondere Hexadecyl-tributyl-phosphoniumchlorid und Tetradecyltributyl-phosphoniumchlorid.

Die antimikrobiellen Wirkstoffe B und C, nämlich (B) quartäre Ammoniumverbindungen sowie (C) Phenole und Biguanidverbindungen, als alleinige antimikrobielle Wirkstoffe in wäßrigen, tensidhaltigen Reinigungsmitteln werden in "Surfactants in Consumer Products" (1987), Seiten 333 bis 340, beschrieben.

Die antimikrobiellen Wirkstoffe A, B und/oder C werden in den erfindungsgemäßen Reinigungsmitteln vorzugsweise in Gewichtsverhältnissen A : B von 8:1 bis 1:8 und A : C nicht unter 1:4 eingesetzt. Die Gesamtmenge an antimikrobiellen Wirkstoffen in den erfindungsgemäßen Reinigungsmitteln beträgt bevorzugt 0,5 Gew.-% bis 15 Gew.-%, insbesondere 1 Gew.% bis 10 Gew.-%.

Weiterhin enthält das erfindungsgemäße Reinigungs- und Desinfektionsmittel ein oder mehrere möglichst schaumarme oberflächenaktive Stoffe, vorzugsweise aus den Gruppen der nichtionischen Tenside und der Amphotenside. Als nichtionische Tenside kommen Alkylglykoside mit Alkylteilen von vorzugsweise 8 bis 22 C-Atomen, Umsetzungsprodukte von 4 bis 20 Molequivalenten Ethylenoxid und/oder Propylenoxid mit Fettalkoholen, Fettsäuren, Fettaminen, deren Fettalkylreste vorzugsweise jeweils 8 bis 22 C-Atome enthalten, und mit Alkylphenolen in Betracht. Auch die endgruppenverschlossener Derivate deratiger Alkoxylierungsprodukte, vorzugsweise mit Endgruppen, die 2 bis 10 C-Atome enthalten, kommen in Frage. Zu derartigen nichtionischen Tensiden gehören beispielsweise die Handelsprodukte Dehypon^{(R)} LS 24, LS 36, LS 45, LS 54, LT 24, LT 104, OCP 502 (Lieferant Henkel), Dehydol^{(R)} LT 30 (Lieferant Henkel), Lutensol^{(R)} LF 224, LT 30 (Lieferant BASF), Triton^{(R)} CF 54 und DF 12 (Lieferant Rohm & Haas). Zu den geeigneten Amphotensiden gehören Derivate tertiärer aliphatischer Amine oder quartärer aliphatischer Ammoniumverbindungen, deren aliphatische Reste geradkettig oder verzweigt sein können und von denen einer eine Carboxy-, Sulfo-, Phosphono, Sulfato- oder Phosphato-Gruppe trägt. Beispiele für derartige Amphotenside sind Dimethyl-tetradecyl-glycin, Dimethyl-hexadecyl-glycin, Dimethyl-octadecyl-glycin, 3-(Dimethyl-dodecylammonio)-1-propansulfonat und die unter den Bezeichnungen Dehyton^{(R)} AB, CB und G (Lieferant Henkel) vertriebenen Amphotenside.

Um unter den Anwendungsbedingungen gute Reinigungsleistungen zu erzielen, sind die Tenside in den erfindungsgemäßen Mitteln vorzugsweise in Mengen von 2 Gew.-% bis 15 Gew.-%, insbesondere von 4 Gew.-% bis 10 Gew.-% vorhanden.

Zusätzlich können die erfindungsgemäßen Reinigungs- und Desinfektionsmittel wasserlösliche organische Lösungsmittel, vorzugsweise aus den Gruppen der Alkohole mit 1 bis 4 C-Atomen, der Glykole mit 2 bis 4 C-Atomen und der aus diesen ableitbaren Diglykole und Diglykolether, enthalten. Derartige Lösungsmittel sind beispielsweise Methanol, Ethanol, Propanol, Isopropanol, Tert.-Butanol, Ethylenglykol, Propylenglykol, Butylenglykol, Diethylenglykol, Dipropylenglykol, Diethylenglykolmonomethylether, Diethylenglykolmonoethylether, Diethylenglykolmonopropylether und Diethylenglykolmonobutylether. Organische Lösungsmittel sind bevorzugt in Mengen nicht über 35 Gew.-%, insbesondere in Mengen von 10 Gew.-% bis 30 Gew.-%, in den erfindungsgemäßen Mitteln enthalten.

Um auch bei Verwendung der erfindungsgemäßen Reinigungsmittelkonzentrate mit hartem Wasser ein gutes Korrosionsverhalten zu gewährleisten, können die erfindungsgemäßen Reinigungs- und Desinfektionsmittel Komplexbildner enthalten. Diese werden vorzugsweise aus den Gruppen der Phosphonsäuren, Hydroxyphosphonsäuren, Aminophosphonsäuren, Phosphonocarbonsäuren, Aminocarbonsäuren und deren Salzen, insbesondere deren Alkalisalzen, ausgewählt. Zu diesen Komplexierungsmitteln gehören beispielsweise Methandiphosphonsäure, Hydroxyethan-1,1-diphosphonsäure, 1-Aminoethan-1,1-diphosphonsäure, Amino-tri(methylenphosphonsäure), Ethylendiamin-tetra(methylenphosphonsäure), Diethylentriamin-penta(methylenphosphonsäure), 2-Phosphonobutan-1,2,4-tricarbonsäure, Nitrilotriessigsäure, Ethylendiamintetraessigsäure und Hydroxyethyl-ethylendiamintriessigsäure. Derartige Korrosionsinhibitoren sind in den erfindungsgemäßen Mitteln vorzugsweise in Mengen nicht über 6 Gew.-%, insbesondere von 1 Gew.-% bis 5 Gew.-%, enthalten.

Darüberhinaus können die erfindungsmäßen Reinigungs- und Desinfektionsmittelkonzentrate in solchen Mitteln übliche Zusätze, wie Farbstoffe, Korrosionsinhibitoren oder Duftstoffe, enthalten. Derartige übliche Zusatzstoffe sind in den erfindungsgemäßen Mitteln bevorzugt in Mengen nicht über 1 Gew.-% vorhanden.

Vorzugsweise enthalten die erfindungsgemäßen Reinigungsmittel die einzelnen Bestandteile in folgenden Mengenanteilen :
0,5 - 15 Gew.-% antimikrobielle Wirkstoffe,
2 - 15 Gew.-% Tensid,
0 - 35 Gew.-% organisches Lösungsmittel,
0 - 6 Gew.-% Komplexbildner,
0 - 1 Gew.-% übliche Zusatzstoffe
Besonders bevorzugt sind wäßrige Reinigungsmittel, die die einzelnen Bestandteile in folgenden Mengenanteilen enthalten :
0,5 - 4,0 Gew.-% Wirkstoff A,
0,5 - 4,0 Gew.-% Wirkstoff B,
0 - 2,0 Gew.-% Wirkstoff C,
4 - 10 Gew.-% Tensid
10 - 30 Gew.-% organisches Lösungsmittel,
1 - 5 Gew.-% Komplexbildner,
0 - 0,5 Gew.-% übliche Zusatzstoffe
Die erfindungsgemäßen Reinigungs- und Desinfektionsmittel-Konzentrate können, falls sie nicht wegen der Natur ihrer Bestandteile einen weitgehend neutralen pH-Wert von vorzugsweise 6,5 bis 7,5 aufweisen, durch Zugabe geringer Mengen üblicher, mit den übrigen Inhaltsstoffen verträglicher Säuren oder Basen, zu denen insbesondere Zitronensäure und Natronlauge gehören, auf pH-Werte in diesem Bereich eingestellt werden.

Die Herstellung der erfindungsgemäßen Reinigungsmittel bietet keine Probleme. Sie erfolgt im einfachsten Fall durch Vermischen ihrer Bestandteile, die in Substanz oder als wäßrige Lösungen eingesetzt werden können.

Das erfindungsgemäße Reinigungs- und Desinfektionsmittel kann als solches oder vorzugsweise in wäßriger Verdünnung, insbesondere in Konzentrationen von 0,5 Gew.-% bis 2 Gew.-%, zur Instrumentendesinfektion oder zur Reinigung und Desinfektion harter Oberflächen verwendet werden. Besonders vorteilhaft wird es in Verfahren in automatisch betriebenen Reinigungsanlagen eingesetzt. Dabei enthält das Verfahren vorzugsweise die Schritte a) Aufsprühen einer wäßrigen, desinfizierenden Reinigungsmittel lösung erhöhter Temperatur, vorzugsweise 50 °C bis 80 °C, die durch Verdünnen des erfindungsgemäßen Konzentrats mit Wasser, vorzugsweise auf Konzentrationen von 0,5 Gew.-% bis 2 Gew.-%, hergestellt worden ist, und gewünschtenfalls b) Aufsprühen einer wäßrigen, gegebenenfalls tensidhaltigen Klarspüllösung sowie gegebenenfalls c) Trocknen, zum Beispiel mit Heißluft. Eine besonders bevorzugte Ausführungsform des Reinigungsverfahrens beinhaltet die Rückführung der gebrauchten, gegebenenfalls mit der Klarspüllösung verdünnten Reinigungsmittel lösung in einen Vorratsbehälter und den erneuten Einsatz der Lösung, der zuvor, gewünschtenfalls durch automatische Zugabe, frisches Reinigungs- und Desinfektionsmittelkonzentrat zudosiert werden kann.

### Beispiele

### Beispiel 1

Durch Zusammengeben und Vermischen der einzelnen Bestandteile wurde ein klares, lagerstabiles Reinigungs- und Desinfektionsmittel (**B1**) folgender Zusammensetzung (Rest auf 100 Gew.-% Wasser) hergestellt :
3 Gew.-% Glutaminsäurederivat^{a)}
1 Gew.-% Dimethyldidecylammoniumpropionat
0,6 Gew.-% Tributyltetradecylphosphoniumchlorid
7 Gew.-% Propoxyliertes C_{12/14}-Fettalkylethoxylat (Dehypon^{(R)} LS, Hersteller Henkel)
2 Gew.-% Diethylenglykolmonobutylether
1,5 Gew.-% 2-Phosphonobutan-1,2,4-tricarbonsäure
3 Gew.-% Trinatriumnitrilotriacetat
30 Gew.-% Isopropanol
Es besaß unverdünnt einen pH-Wert von 6,9 und wies in Leitungswasser in Konzentrationen von 1,0 Gew.-% oder 0,5 Gew.-% einen pH-Wert von jeweils 7,0 auf.
^{a)} Produkt der Umsetzung von Glutaminsäure mit N-C_{12/14}-Fettalkyl-propylendiamin (molares Verhältnis 1:1) unter Austritt von 1 Molequivalent Wasser gemäß EP-B-156 275.

### Beispiel 2: Praxistest

Das Mittel **B1** wurde in einer automatischen Bettenwaschanlage (Hersteller Münchener Medizin Mechanik) in Anlehnung an die Richtlinie des Bundesgesundheitsamtes zur Prüfung von thermischen Desinfektionsverfahren in Reinigungsautomaten (Bundesgesundheitsblatt 20 (1980), 356) und die Richtlinien der Deutschen Gesellschaft für Hygiene und Mikrobiologie für die Prüfung und Bewertung chemischer Desinfektionsverfahren (Zbl. Bakt. Hyg., I. Abt. Orig. B 165 (1977), 335) auf desinfizierende Wirksamkeit geprüft. Die Untersuchungen wurden an Krankenhausbetten mit Flachrost-Liegeflächen, kunstoffbeschichteten Metallrahmen und Kopf- und Fußteilen aus Resopal^{(R)} mit matter Oberfläche vorgenommen. Als Testkeim wurde Streptococcus faecalis ATTC 6057 mit kontrollierter Thermoresistenz (70 °C/10 Minuten) verwendet; als Trägermedien wurden defibriniertes Hammelblut (Hersteller Oxoid) und Kunstsputum aus Mucin (Hersteller Difco), Traganth (Hersteller Merck) und Rinderserum (Hersteller Oxoid) eingesetzt. Die Keimdichte in den Trägermedien betrug 4,4 · 10⁸ pro Mililiter (Blut) beziehungsweise 3,8 · 10⁸ pro Mililiter (Kunstsputum). Zur Kontamination wurden Resopal^{(R)}-Platten (15 cm x 15 cm x 1,5 mm) mit matter Oberfläche und Edelstahlplatten (8 cm x 8 cm x 1,5 mm) mit polierter Oberfläche verwendet, auf denen Testfelder von jeweils 5 cm x 5 cm mit wasserfester Farbe markiert wurden. Jedes Testfeld wurde mittels steriler Tupfer mit einer gleichmäßigen Schicht aus jeweils etwa 0,2 ml Testanschmutzung versehen, die man 24 Stunden trocknen ließ. Die Testplatten wurden an allen Rändern mit transparentem Klebeband an oben beschriebenen Krankenhausbetten derart befestigt, daß von jedem Testmaterial und jeder Testanschmutzung eine Platte an Kopf- und Fußteil, jeweils an Außen- und Innenseite, vorhanden war. Jedes Bett war folglich mit insgesamt 16 Prüffeldern kontaminiert.

Die Betten wurden mit Umlaufwasser (Temperatur 65 °C), das 1,5 Gew.-% des Reinigungsmittels **B1** enthielt, gereinigt, wobei vor Versuchsbeginn (5 Betten) und zwischen den einzelnen Testbetten (jeweils 1 bis 3 Betten) Leerchargen liefen. Zwischen zwei Testbetten erfolgte jeweils eine Zugabe von je 5 ml Hammelblut in den Reinigungslaugen-Vorratsbehälter. Nach jedem Testbett wurde 1 Probe, ab dem 5. Bett 2 Proben (jeweils 100 ml) aus der Reinigungslauge genommen und nach Zugabe von 100 ml einer konzentrierten Nährstoffbouillon (Lieferant Merck) und Bebrütung (14 Tage bei 37 °C) mikrobiologisch untersucht (Tabelle 3).

Die Einwirkzeit der Reinigungslösung auf die kontaminierten Betten betrug 90 Sekunden oder 60 Sekunden (Tabellen 1 und 2), danach wurde mit einer 0,05 gew.-prozentigen wäßrigen Lösung eines pH-neutralen Netzmittels aus alkoxylierten Fettalkoholen (Dehypon^{(R)} LS 54, Lieferant Henkel) klargespült (30 Sekunden), wobei die Klarspüllösung in die umgewälzte Reinigungslösung

**Tabelle 1**

| (Keimträger Blut) | | | | | | | | | | | | | | | | | |
|---|---|---|---|---|---|---|---|---|---|---|---|---|---|---|---|---|---|
| | | I | | | | II | | | | III | | | | IV | | | |
| | | E | | R | | E | | R | | E | | R | | E | | R | |
| DL | EWZ | a | b | a | b | a | b | a | b | a | b | a | b | a | b | a | b |
| 6 | 90 | - | - | - | - | - | - | - | - | - | - | - | - | - | - | - | - |
| 10 | 90 | + | + | - | - | - | - | - | - | - | - | - | - | - | - | - | - |
| 14 | 90 | + | - | - | + | - | + | - | - | - | - | - | - | - | - | - | - |
| 18 | 90 | - | - | - | - | - | - | - | - | nd | | - | - | nd | | - | - |
| 21 | 90 | nd | | - | - | nd | | - | - | nd | | - | - | nd | | - | - |
| 24 | 60 | - | - | - | - | - | + | - | - | - | - | - | - | - | - | - | - |
| 26 | 60 | - | - | - | - | - | - | - | - | - | - | - | - | - | - | - | - |
| 28 | 60 | - | - | - | - | - | - | - | - | - | - | - | - | - | - | - | - |
| 30 | 60 | - | - | - | - | - | - | - | - | nd | | - | - | nd | | - | - |
| 32 | 60 | nd | | - | - | nd | | - | - | nd | | - | - | nd | | - | - |

**Tabelle 2**

| (Keimträger Kunstsputum) | | | | | | | | | | | | | | | | | |
|---|---|---|---|---|---|---|---|---|---|---|---|---|---|---|---|---|---|
| | | I | | | | II | | | | III | | | | IV | | | |
| | | E | | R | | E | | R | | E | | R | | E | | R | |
| DL | EWZ | a | b | a | b | a | b | a | b | a | b | a | b | a | b | a | b |
| 6 | 90 | - | - | - | - | - | - | - | - | - | - | - | - | - | - | - | - |
| 10 | 90 | - | - | - | - | - | - | - | - | - | - | - | - | - | - | - | - |
| 14 | 90 | - | - | - | - | - | - | - | - | - | - | - | - | - | - | - | - |
| 18 | 90 | - | - | - | + | - | - | - | - | nd | | - | - | nd | | - | - |
| 21 | 90 | nd | | - | - | nd | | - | - | nd | | - | - | nd | | - | - |
| 24 | 60 | - | - | - | - | - | - | - | - | - | - | - | - | - | - | - | - |
| 26 | 60 | - | - | - | - | - | - | - | - | - | - | - | - | - | - | - | - |
| 28 | 60 | - | - | - | - | - | - | - | - | - | - | - | - | - | - | - | - |
| 30 | 60 | - | - | - | - | - | - | - | - | nd | | - | - | nd | | - | - |
| 32 | 60 | nd | | - | - | nd | | - | - | nd | | - | - | nd | | - | - |
| Legende zu den Tabellen 1 und 2 : DL : Durchlaufzahl EWZ: Einwirkzeit [Sekunden] E: Testfläche aus Edelstahl R: Testfläche aus Resopal^{(R)} I: Testfläche an Kopfteil außen befestigt II: Testfläche Kopfteil innen befestigt III: Testfläche Fußteil außen befestigt IV: Testfläche Fußteil innen befestigt a: Probennahme mit Rodac^{(R)}-Platte b: Probennahme mit Tupfer nd: Bestimmung nicht durchgeführt | | | | | | | | | | | | | | | | | |

gelangte, und anschließend getrocknet (180 Sekunden). Von den einzelnen Testfeldern wurden vor und nach dem Reinigen mit sterilen Tupfern oder mit Rodac ^{(R)}-Platten und von diesen mit sterilen Tupfern Proben genommen, die Tupfer auf feste Nährböden ausgestrichen und diese nach Inkubation von jeweils 72 Stunden bei 37 °C mikroskopisch auf den eingesetzten Testkeim untersucht.

Vor Versuchsbeginn konnten die Testkeime in allen Proben von allen Testfeldern nachgewiesen werden (rasenförmiges Wachstum auf den Nährböden). Nach dem maschinellen Reinigen und Desinfizieren waren alle Testfelder optisch sauber. Bei 6 Testfeldern zeigte sich unter dem transparenten Klebeband ein Sprühschatten mit Kontaminationsresten. Keime auf den gewaschenen Testfeldern waren mit 7 Ausnahmen nicht nachweisbar (Tabellen 1 und 2). Aus den entnommenen Reinigungslauge-Proben konnte der Testkeim nicht reisoliert werden (Tabelle 3).

**Tabelle 3**

| Mikrobiologische Untersuchung der Reinigungslauge | | |
|---|---|---|
| Probe nach Bett Nr. | Wachstum in Probe 1 | Wachstum in Probe 2 |
| 1 | - | Spb |
| 2 | - | - |
| 3 | - | - |
| 4 | - | nd |
| 5 | - | nd |
| 6 | - | nd |
| 7 | - | nd |
| 8 | - | - |
| 9 | Spb | - |
| 10 | - | - |
| Legende zu Tabelle 3 : - : Kein aerobes Wachstum Spb : Aerobe Sporenbildner nd : Bestimmung nicht durchgeführt | | |

## Patentansprüche

1. Wäßriges tensidhaltiges Reinigungs- und Desinfektionsmittelkonzentrat mit einem Gehalt eines Wirkstoffes A, der durch Umsetzung eines N-substituierten Propylendiamins nach Formel (I),
R¹-NH-(CH₂)₃-NH₂ (I),
in der R¹ für einen Alkylrest mit 8 bis 18 C-Atomen, vorzugsweise 12 bis 14 C-Atomen, steht, mit Glutaminsäure oder Glutaminsäurederivaten gemäß Formel (II),
R²-O-CO-(CH₂)₂-CH(NH₂)-COOH (II),
in der R² Wasserstoff oder einen Alkylrest mit 1 bis 4 Kohlenstoffatomen bedeutet, und gegebenenfalls Umsetzung des so erhaltenen Produkts mit Ethylenoxid und/oder Propylenoxid und gegebenenfalls weitere Umsetzung mit organischen oder anorganischen Säuren erhältlich ist, dadurch gekennzeichnet, daß es weitere antimikrobielle Wirkstoffe B und/oder C enthält, wobei B eine quartäre Ammoniumverbindung nach Formel (III), 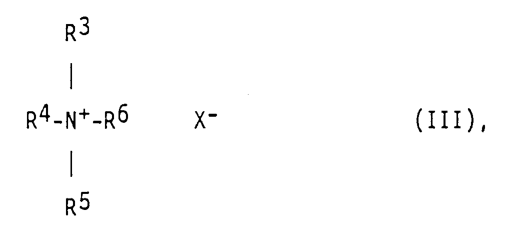 in der R³ und R⁴ unabhängig voneinander Alkylreste mit jeweils 1 bis 3 C-Atomen oder Benzylreste, halogenierte oder alkylierte Benzylreste, R⁵ und R⁶ unabhängig voneinander Alkyl- oder Benzyl- oder halogenierte oder alkylierte Benzylreste mit jeweils 6 bis 22 C-Atomen und X⁻ ein Anion vorzugsweise aus den Gruppen der Halogenide oder Carboxylate bedeuten, und C einen weiteren antimikrobiellen Wirkstoff aus der Gruppe der Phenole, Biguanidverbindungen oder der quartären Phosphoniumverbindungen nach Formel (IV) darstellt, 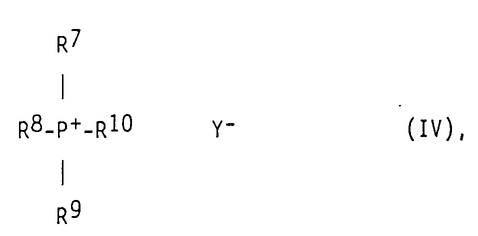 in der R⁷, R⁸, R⁹ und R¹⁰ unabhängig voneinander Alkyl-, Alkenyl-, Aryl- oder Alkylarylreste mit jeweils 1 bis 16 C-Atomen und Y⁻ ein Anion vorzugsweise aus den Gruppen der Halogenide oder Carboxylate, insbesondere Acetat oder Propionat, bedeuten.

2. Reinigungs- und Desinfektionsmittel nach Anspruch 1, dadurch gekennzeichnet, daß es die antimikrobiellen Wirkstoffe A und B in Gewichtsverhältnissen von 8:1 bis 1:8 und die antimikrobiellen Wirkstoffe A und C in Gewichtsverhältnissen von nicht unter 1:4 enthält.

3. Reinigungs- und Desinfektionsmittel nach einem der Ansprüche 1 oder 2, dadurch gekennzeichnet, daß es eine Kombination der antimikrobiellen Wirkstoffe A, B und C (IV) enthält.

4. Reinigungs- und Desinfektionsmittel nach einem der Ansprüche 1 bis 3, dadurch gekennzeichnet, daß die darin enthaltenen Tenside aus der Gruppe umfassend die nichtionischen Tenside, insbesondere die ethoxylierten und/oder propoxylierten Fettalkohole, Fettsäure, Fettamine und Alkylphenole, die Amphotenside, insbesondere die tertiären oder quartären aliphatischen Amine, deren aliphatische Reste geradkettig oder verzweigt sind und von denen einer eine Carboxy-, Sulfo-, Phosphono-, Sulfato- oder Phosphato-Gruppe trägt, sowie deren Gemische ausgewählt werden.

5. Reinigungs- und Desinfektionsmittel nach einem der Ansprüche 1 bis 4, dadurch gekennzeichnet, daß es zusätzlich einen Komplexbildner aus der Gruppe umfassend die Phosphonsäuren, Hydroxyphosphonsäuren, Phosphonocarbonsäuren, Aminocarbonsäuren, Aminophosphonsäuren und deren Salze, insbesondere deren Alkalisalze sowie deren Gemische, enthält.

6. Reinigungs- und Desinfektionsmittel nach einem der Ansprüche 1 bis 5, dadurch gekennzeichnet, daß es zusätzlich ein wasserlösliches organisches Lösungsmittel, ausgewählt aus der Gruppe umfassend die Alkohole mit 1 bis 4 C-Atomen, die Glykole mit 2 bis 4 C-Atomen und die aus diesen ableitbaren Diglykole und Diglykolether sowie deren Gemische, enthält.

7. Wäßriges Reinigungs- und Desinfektionsmittel nach einem der Ansprüche 1 bis 6, dadurch gekennzeichnet, daß es
0,5 - 15 Gew.-% antimikrobielle Wirkstoffe,
2 - 15 Gew.-% Tensid,
0 - 35 Gew.-% organisches Lösungsmittel,
0 - 6 Gew.-% Komplexbildner
0 - 1 Gew.-% übliche Zusatzstoffe
enthält.

8. Wäßriges Reinigungs- und Desinfektionsmittel nach Anspruch 7, dadurch gekennzeichnet, daß es
0,5 - 4,0 Gew.-% Wirkstoff A,
0,5 - 4,0 Gew.-% Wirkstoff B,
0 - 2,0 Gew.-% Wirkstoff C,
4 - 10 Gew.-% Tensid,
10 - 30 Gew.-% organisches Lösungsmittel,
1 - 5 Gew.-% Komplexbildner,
0 - 0,5 Gew.-% übliche Zusatzstoffe
enthält.

9. Reinigungs- und Desinfektionsmittel nach einem der Ansprüche 1 bis 8, dadurch gekennzeichnet, daß es in 1 gewichtsprozentiger wäßriger Lösung einen pH-Wert von 6,8 bis 7,2 aufweist und daß dieser pH-Wert gegebenenfalls durch Zugabe mit den sonstigen Inhaltsstoffen verträglicher Säuren oder Basen, insbesondere Zitronensäure oder Natronlauge, erreicht wird.

10. Verfahren zur Sprüh-Reinigung und -Desinfektion von Gegenständen aus medizinischen Einrichtungen in automatisch betriebenen Anlagen, enthaltend die Schritte a) Aufsprühen einer wäßrigen, desinfizierenden Reinigungsmittellösung erhöhter Temperatur, die durch Verdünnen eines Reinigungskonzentrats nach einem der Ansprüche 1 bis 9 hergestellt worden ist, und gewünschtenfalls b) Aufsprühen einer wäßrigen, gegebenenfalls tensidhaltigen Klarspüllösung sowie gewünschtenfalls c) Trocknen, vorzugsweise mittels Heißluft.

11. Verfahren nach Anspruch 10, dadurch gekennzeichnet, daß die automatische Anlage nach dem Umlaufprinzip arbeitet und die desinfizierende Reinigungsmittellösung nach Gebrauch, gegebenenfalls mit der Klarspüllösung verdünnt, zurückgeführt wird.

12. Verfahren nach einem der Ansprüche 10 oder 11, dadurch gekennzeichnet, daß die Konzentration des Reinigungs- und Desinfektionsmittels in der Reinigungslösung 0,5 Gew.-% bis 2 Gew.-% beträgt.

## Claims

1. A water-based, surfactant-containing concentrated cleaning and disinfecting preparation containing an antimicrobial agent **A** obtainable by reaction of an N-substituted propylenediamine corresponding to formula (I)
R¹-NH-(CH₂)₃-NH₂ (I)
in which R¹ is an alkyl radical containing 8 to 18 carbon atoms and preferably 12 to 14 carbon atoms,
with glutamic acid or glutamic acid derivatives corresponding to formula (II)
R²-O-CO-(CH₂)₂-CH(NH₂)-COOH (II)
in which R² is hydrogen or a C₁₋₄ alkyl radical,
and optionally reaction of the product thus obtained with ethylene oxide and/or propylene oxide and, optionally, further reaction with organic or inorganic acids,
characterized in that it contains other antimicrobial agents **B** and/or **C**,
**B** being a guaternary ammonium compound corresponding to formula (III) 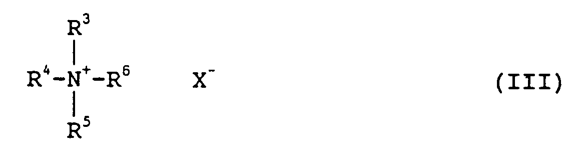 in which R³ and R⁴ independently of one another represent alkyl radicals containing 1 to 3 carbon atoms or benzyl radicals, halogenated or alkylated benzyl radicals, R⁵ and R⁶ independently of one another represent alkyl or benzyl radicals or halogenated or alkylated benzyl radicals containing 6 to 22 carbon atoms and X⁻ is an anion, preferably from the groups of halides or carboxylates,
and **C** being another antimicrobial agent from the group of phenols, biguanide compounds or quaternary phosphonium compounds corresponding to formula (IV) 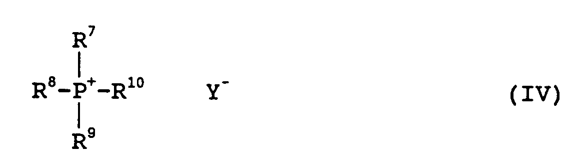 in which R⁷, R⁸, R⁹ and R¹⁰ independently of one another represent alkyl, alkenyl, aryl or alkylaryl radicals containing 1 to 16 carbon atoms and Y⁻ is an anion, preferably from the groups of halides and carboxylates, more particularly acetate or propionate.

2. A cleaning and disinfecting preparation as claimed in claim 1, characterized in that it contains the antimicrobial agents **A** and **B** in ratios by weight of 8:1 to 1:8 and antimicrobial agents **A** and **C** in ratios by weight of no lower than 1:4.

3. A cleaning and disinfecting preparation as claimed in claim 1 or 2, characterized in that it contains a combination of antimicrobial agents **A**, **B** and **C** (IV).

4. A cleaning and disinfecting preparation as claimed in any of claims 1 to 3, characterized in that the surfactants present therein are selected from the group consisting of nonionic surfactants, more particularly ethoxylated and/or propoxylated fatty alcohols, fatty acids, fatty amines and alkylphenols, amphoteric surfactants, more particularly tertiary or guaternary aliphatic amines, of which the aliphatic radicals are linear or branched and one of which bears a carboxy, sulfo, phosphono, sulfato or phosphato group, and mixtures thereof.

5. A disinfecting and cleaning preparation as claimed in any of claims 1 to 4, characterized in that it also contains a complexing agent from the group consisting of phosphonic acids, hydroxyphosphonic acids, phosphonocarboxylic acids, aminocarboxylic acids, aminophosphonic acids and salts thereof, more particularly alkali metal salts and mixtures thereof.

6. A cleaning and disinfecting preparation as claimed in any of claims 1 to 5, characterized in that it also contains a water-soluble organic solvent selected from the group consisting of C₁₋₄ alcohols, C₂₋₄ glycols and the diglycols and diglycol ethers derivable therefrom and also mixtures thereof.

7. A water-based cleaning and disinfecting preparation as claimed in any of claims 1 to 6, characterized in that it contains
0.5 to 15 % by weight antimicrobial agents
2 to 15 % by weight surfactant,
0 to 35 % by weight organic solvent,
0 to 6 % by weight complexing agent,
0 to 1 % by weight typical additives.

8. A water-based cleaning and disinfecting preparation as claimed in claim 7, characterized in that it contains
0.5 to 4.0% by weight antimicrobial agent **A**,
0.5 to 4.0% by weight antimicrobial agent **B**,
0 to 2.0% by weight antimicrobial agent **C**,
4 to 10 % by weight surfactant.
10 to 30 % by weight organic solvent,
1 to 5 % by weight complexing agent,
0 to 0.5% by weight typical additives.

9. A cleaning and disinfecting preparation as claimed in any of claims 1 to 8, characterized in that, in the form of a 1% by weight aqueous solution, it has a pH value of 6.8 to 7.2 and in that this pH value is optionally adjusted by addition of acids or bases compatible with the other ingredients, more particularly citric acid or sodium hydroxide.

10. A process for the spray cleaning and disinfection of items of medical equipment in automatic machines comprising the steps of a) spraying on an aqueous disinfecting cleaning solution of elevated temperature which has been prepared by dilution of the concentrate claimed in any of claims 1 to 9; if desired, b) spraying on an aqueous optionally surfactant-containing clear rinse solution and, optionally, c) drying, preferably with hot air.

11. A process as claimed in claim 10, characterized in that the automatic machine operates on the circulation principle and the disinfecting cleaning solution is returned after use, optionally diluted with the clear rinse solution.

12. A process as claimed in claim 10 or 11, characterized in that the concentration of the cleaning and disinfectant in the cleaning solution is from 0.5% by weight to 2% by weight.

## Revendications

1. Concentré aqueux d'agent de nettoyage et de désinfection renfermant des tensioactifs, avec une concentration en substance active A, qui est obtenable par réaction d'une propylènediamine N-substituée de formule (I),
R¹-NH-(CH₂)₃-NH₂ (I),
dans laquelle R¹ représente un radical alkyle comportant 8 à 18 atomes de C, de préférence 12 à 14 atomes de C, avec de l'acide glutamique ou des dérivés de l'acide glutamique de formule (II),
R²-O-CO-(CH₂)₂-CH(NH₂)-COOH (II),
dans laquelle R² représente l'hydrogène ou un radical alkyle comportant 1 à 4 atomes de carbone et, le cas échéant, réaction du produit ainsi obtenu avec de l'oxyde d'éthylène et/ou de l'oxyde de propylène et, le cas échéant, réaction ultérieure avec des acides organiques ou inorganiques, caractérisé en ce qu'il renferme d'autres substances actives antimicrobiennes B
et/ou C, B étant un composé quaternaire de l'ammonium de formule (III), 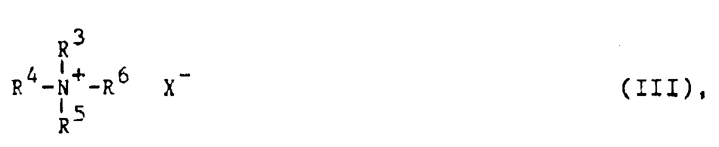 dans laquelle R³ et R⁴ représentent indépendamment l'un de l'autre des radicaux alkyle comportant chacun 1 à 3 atomes de C, ou des groupes benzyle ou des groupes benzyle halogénés ou alkylés, R⁵ et R⁶ correspondent indépendamment l'un de l'autre à des groupes alkyle ou benzyle ou à des groupes benzyle halogénés ou alkylés comportant chacun 6 à 22 atomes de C, tandis que X⁻ est un anion appartenant de préférence aux groupes des halogénures ou des carboxylates, et C étant une autre substance active antimicrobienne appartenant au groupe des phénols, des biguanides ou des composée de phosphonium quaternaires de formule (IV), 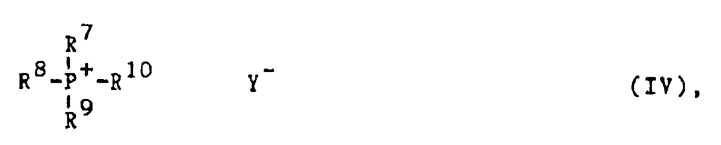 dans laquelle R⁷, R⁸, R⁹ et R¹⁰ représentent indépendamment l'un de l'autre des radicaux alkyle, alcényle, aryle ou alkylaryle comportant chacun 1 à 16 atomes de C, tandis que Y⁻ est un anion, appartenant de préférence aux groupes des halogénures ou des carboxylates, en particulier un acétate ou un propionate.

2. Agent de nettoyage et de désinfection selon la revendication 1, caractérisé en ce qu'il renferme les substances actives antimicrobiennes A et B en rapports pondéraux de 8:1 à 1:8, et les substances actives antimicrobiennes A et C, en rapports pondéraux non inférieurs à 1:4.

3. Agent de nettoyage et de désinfection selon une des revendications 1 ou 2, caractérisé en ce qu'il renferme une association des substances actives antimicrobiennes A , B et C (IV).

4. Agent de nettoyage et de désinfection selon une de revendications 1 à 3, caractérisé en ce que les tensioactifs y contenus sont sélectionnés parmi le groupe comprenant les surfactifs non ioniques, en particulier les acides gras, les amines grasses, les alkylphénols et les alcools gras éthoxylés et/ou propoxylés, et les tensioactifs amphotères, en particulier les amines aliphatiques tertiaires ou quaternaires, dont les radicaux aliphatiques sont à chaîne droite ou ramifiée et dont un porte un groupe carboxy, sulfo, phosphono, sulfato ou phosphato, ainsi que des mélanges de ceux-ci.

5. Agent de nettoyage et de désinfection selon une des revendications 1 à 4, caractérisé en ce qu'il renferme en plus un agent complexant appartenant au groupe formé des acides phosphoniques, hydroxyphosphoniques, phosphonocarboxyliques, aminocarboxyliques, aminophosphoniques et de leurs sels, en particulier leurs sels de métaux alcalins ainsi que des mélanges de ceux-ci.

6. Agent de nettoyage et de désinfection selon une des revendications 1 à 5, caractérisé en ce qu'il renferme en plus un solvant organique soluble dans l'eau, sélectionné parmi le groupe formé des alcools comportant 1 à 4 atomes de C, des glycols possédant 2 à 4 atomes de C, et des diglycols et diglycoléthers dérivables de ceux-ci, ainsi que des mélanges de ceux-ci.

7. Agent de nettoyage et de désinfection aqueux selon une des revendications 1 à 6, caractérisé en ce qu'il renferme
0,5 à 15 % en poids de substances actives antimicrobiennes,
2 à 15 % en poids de tensioactif,
0 à 35 % en poids de solvant organique
0 à 6 % en poids d'agent complexant,
0 à 1 % en poids d'additifs usuels.

8. Agent de nettoyage et de désinfection aqueux selon la revendication 7, caractérisé en ce qu'il renferme
0,5 à 4,0 % en poids de substance active A,
0,5 à 4,0 % en poids de substance active B,
0 à 2,0 % en poids de substance active C,
4 à 10 % en poids de tensioactif,
10 à 30 % en poids de solvant organique,
1 à 5 % en poids d'agent complexant,
0 à 0,5 % en poids d'additifs usuels.

9. Agent de nettoyage et de désinfection selon une des revendications 1 à 8, caractérisé en ce qu'il présente en solution aqueuse à 1 pour cent on poids un pH compris entre 6,8 et 7,2 et que ledit pH est atteint, le cas échéant, par adjonction d'acides ou de bases compatibles avec les autres constituants, en particulier l'acide citrique ou la soude caustique.

10. Procédé de nettoyage et de désinfection par pulvérisation d'objets d'équipement médicaux dans des installations automatiques, comprenant les étapes a) pulvérisation d'une solution aqueuse désinfectante d'agent nettoyant à température accrue, qui a été préparée par dilution d'un concentré d'agent nettoyant selon une des revendications 1 à 9 et, le cas échéant, b) pulvérisation d'une solution aqueuse de rinçage finale contenant éventuellement des tensioactifs, ainsi que, le cas échéant, c) séchage, de préférence à l'air chaud.

11. Procédé selon la revendication 10, caractérisé en ce que l'installation automatique fonctionne selon le principe de circulation et en ce que la solution désinfectante d'agent nettoyant est recyclée après usage, le cas échéant avec la solution de rinçage final.

12. Procédé selon une des revendications 10 ou 11, caractérisé on ce que la concentration de l'agent de nettoyage et de désinfection dans le solution de nettoyage atteint 0,5 à 2 % en poids.
